# EUROPEAN PATENT SPECIFICATION

(11) **EP 1 960 509 B1**
(45) Date of publication and mention of the grant of the patent: **24.10.2018**
(21) Application number: 06808702.2
(22) Date of filing: 28.11.2006
(51) Int. Cl.: C12M 1/34, C09K 8/58

(54) **METHOD OF CULTURING UNICELLULAR ORGANISMS**
VERFAHREN ZUR KULTIVIERUNG EINZELLIGER ORGANISMEN
PROCÉDÉ DE CULTURE D'ORGANISMES UNICELLULAIRES

(30) Priority: 28.11.2005 GB 0524193
(43) Date of publication of application: 27.08.2008
(73) Proprietor: Statoil Petroleum AS, 4035 Stavanger (NO)
(72) Inventor: KOTLAR, Hans, Kristian, N-7072 Heimdal (NO); BRAKSTAD, Odd, Gunnar, N-7014 Trondheim (NO); WINNBERG, Asgeir, N-7092 Tiller (NO); MARKUSSEN, Sidsel, N-7089 Heimdal (NO)
(74) Representative: Gordon, Kirsteen Helen
(86) International application number: PCT/GB2006/004443
(87) International publication number: WO 2007/060473

(56) References cited:
- WO-A-01/68904
- WO-A-02/095187
- WO-A-2005/115648
- US-A1- 5 492 828
- VANCE I ET AL: "An improved sampling technique for bacterial cultures under high hydrostatic pressure", JOURNAL OF MICROBIOLOGICAL METHODS, ELSEVIER, AMSTERDAM, NL, vol. 4, no. 1, 1 June 1985 (1985-06-01), pages 37-43, XP023699192, ISSN: 0167-7012, DOI: 10.1016/0167-7012(85)90006-5 [retrieved on 1985-06-01]

## Description

This invention relates to a method of culturing unicellular organisms, in particular prokaryotes, e.g. eubacteria and archaea. We additionally describe apparatus therefor, uses and inhibition of such organisms in subterranean applications, to novel such organisms, and to compositions and libraries containing them.
Natural subterranean hydrocarbon reservoirs provide a finite source of oil and gas. It is thus important to optimize hydrocarbon recovery from such reservoirs. Many techniques are used for this purpose but there is a continuing need for new techniques.
To many people's surprise, the rock which is impregnated with the hydrocarbon in such reservoirs, even though it maybe thousands of metres below the surface and thus at very high temperatures and pressures, is not sterile. Unicellular organisms, in particular prokaryotes such as eubacteria and archaea, are present. It has even been proposed that such microorganisms may have been involved in the production of the hydrocarbon. WO 01/68904 describes a process for stimulating the activity of microbial consortia in a hydrocarbon-bearing subterranean formation to convert the hydrocarbons to methane, the method comprising analysing and detecting the presence of microbial consortia in the subterranean formation, determining the characteristics of one or more microorganisms of the consortia, using that information to determine an ecological environment and modifying the formation environment.
We have realised that promoting or inhibiting the growth of such endogenous microorganisms, or introducing cultures of microorganisms isolated from subterranean rock formations, provides a means of improving reservoir management. In order to do this however such microorganisms must be collected and cultured, and if appropriate challenged so that means for growth inhibition may be found.
While some microorganisms retrieved from subterranean rock formations have been cultured at ambient surface conditions, we have surprisingly found that the range of microorganisms that may be isolated and tested is significantly greater if culturing is effected under physicochemical conditions resembling those of the reservoir itself, and in particular if samples taken from a reservoir are maintained at elevated pressure before and during culturing.

This is particularly surprising since liquids, unlike gases, are only very poorly compressible. Thus a bacterium in a liquid culture medium is essentially a liquid filled membrane within a surrounding liquid with negligible, if any, pressure differential across the membrane. It would therefore be expected that bacterial growth would be essentially unaffected by the pressure of the culture medium. This turns out not to be the case however as we have found that culturing hydrocarbon samples under high pressure and under ambient pressure results in growth of quite different organisms and in particular some organisms growing under high pressure conditions simply do not grow at ambient (i.e. earth surface) pressure. Moreover, certain of the enzymes used by these piezophilic organisms do not seem to function at ambient pressure, presumably due to differences in tertiary structure. Vance & Richmond (1985) describe a sampling technique for bacterial cultures subjected to high hydrostatic pressure. Vance & Richmond describe a sample-receiving vessel with a motor driven interface. By precisely matching the pressure in the bulk culture and the sample-receiving vessel none of the sample is subjected to the high shear forces common to other designs of high pressure samplers.

Thus while it has been known to culture individual subterranean microorganisms, it has not previously been known to culture a subterranean ecosystem consisting of a multiplicity of microorganisms some of which do not thrive under surface conditions. If the effects of a down-hole treatment are to be reproduced in laboratory conditions, then it is necessary to use the new method of this invention.

Thus viewed from one aspect the invention provides a method of producing a culture of subterranean microorganisms, said method comprising:
(i) obtaining a flow of hydrocarbon fluid containing microorganisms from a subterranean reservoir and taking a sample of said fluid after the flow has left the ground, wherein the pressure of the fluid flow at the point of sampling exceeds ambient pressure; and
(ii) while maintaining said sample at the pressure of the fluid flow at the point of sampling, transferring it into a fermentation reactor;
(iii) incubating said sample in said reactor at a pressure of 50-150% of that of the reservoir from which the sample was taken,
such that the conditions in the fermentation reactor promote culturing of the microorganisms contained within the hydrocarbon fluid sample.
The sample will typically be incubated at a pressure of 100 to 900 bar, preferably 230 to 280 bar. The pressure should correspond to a pressure of 50 to 150% of that of the reservoir from which it was taken or of that of the formation which the cultured microorganism is intended to treat, more especially 80% to 120%, particularly 90 to 110%.
The sample is preferably a liquid hydrocarbon sample; however aqueous samples may also be used. Samples taken from reservoirs generally include gases, lipids, organic acids, etc.

The incubation temperature will typically be 60 to 160°C, preferably 80 to 100°C, especially preferably within 20°C of that of the reservoir from which the sample was taken or that of the formation which the cultured microorganism is intended to treat, especially within 10°C. Incubation is preferably effected by mixing the sample, before, during or after transfer to the fermentation reactor, with a nutrient. The nutrient may comprise minerals and/or vitamins but preferably comprises at least pulverulent rock, especially preferably of rock from the reservoir from which the sample was taken or from the formation that the cultured microorganism is intended to treat, or rock geologically comparable to such reservoir rock. If desired the nutrient will also comprise fluid from the reservoir from which the sample is taken. In general, the reservoir fluid will provide the carbon source for microorganism growth. Generally, as fermentation progresses, different nutrients or inhibitors may be added to determine their effects on microbial growth.
Incubation in the method of the invention will typically be for from 7 to 360 days, especially 180 to 240 days. If desired incubation may be monitored and controlled so as to be terminated if microorganism growth has not occurred or if it has reached a pre-set acceptable level. Monitoring may be done in situ or on material extracted from the fermentation reactor and may be effected in any suitable fashion. Thus, for example, sample turbidity, uptake of radioactive tracers in nutrient material, organic solids content, etc. may typically be used as parameters to be monitored. Monitoring may typically be effected by drawing off aliquots of the culture medium and subjecting these to analysis by GC, LC-MS, MS, etc., e.g. to show gas generation or consumption or lipid profiles.

Once sufficient microorganism growth has taken place, it will generally be desirable to identify the microorganisms that have proliferated in the culture. This can readily be done by cell lysis followed by nucleic acid fragmentation (e.g. using DNAses or RNAses), fragment replication (e.g. using PCR with primers universal to prokaryotes or groups thereof); and fragment separation (e.g. using gel electrophoresis). The fragment "signature" may then be compared against databases of prokaryote nucleic acid fragments in order to determine which microorganisms are present in the culture and whether any of these are novel. Where novel microorganisms are found, it will generally be desirable to isolate and possibly also sequence them. Isolation may typically be effected by dilution followed by incubation, i.e. using conventional techniques but still incubating under elevated pressure. Sequencing may be effected by conventional techniques.

Where a viable sample of an individual microorganism or the combination of microorganisms is desired, this maybe achieved by bringing the culture to ambient pressure and temperature very slowly, e.g. over at least 10 hours, preferably at least 24 hours. If desired, during the depressurization, the culture may be exposed to bursts of electrical field to cause electroporosis and hence relieve the pressure difference across the cellular membrane.

The microorganisms may then be separated from the culture by conventional means, e.g. centrifugation, resuspension in a sterile fluid, re-centrifugation, etc. The resulting material may then if desired be lyophilized for storage and/or transport. The fluids used will typically be mineral oil or glycerol.

More preferably, however, samples of the culture maybe stored in pressurized containers, e.g. at the pressure under which incubation took place.

We also describe a pressurized container, e.g. having an internal pressure of 100 to 900 bar, especially 200 to 350 bar, containing therein a microorganism, optionally in a hydrocarbyl fluid (e.g. glycerol or mineral oil), and preferably provided with a valved sampling port. We further describe a microorganism library comprising a plurality of such pressurized containers, e.g. at least 10, preferably at least 100. Storing the culture under pressure but at ambient or sub-ambient temperature (e.g. down to liquid nitrogen temperature) is viable since subterranean microorganisms, under such conditions, may be essentially dormant.

The content of the mixture being incubated may be varied to mimic different subterranean conditions. Thus for example sulphate, salt, water, methane, nitrogen, and carbon dioxide contents may be varied to imitate conditions encountered as water penetration into the hydrocarbon bearing strata occurs, or those conditions when water, natural gas, nitrogen or carbon dioxide are injected into the reservoir to enhance hydrocarbon recovery, or those conditions encountered when a squeeze is applied to a producer well to effect a down-hole well treatment (e.g. to administer a scale inhibitor). In this way, from the culture proliferation during incubation, it is possible to determine conditions suited to microorganism growth; conditions suitable for suppression of microorganism growth; appropriate microorganisms for injection down-hole to produce biomass (e.g. to hinder water flow); etc. With such factors determined, it is then possible to effect a down-hole treatment designed to enhance or suppress microorganism growth.
Viewed from a further aspect therefore the invention provides a process for subterranean reservoir treatment, e.g. a process for treatment of a hydrocarbon well, which process comprises injecting a culture of a microorganism into said reservoir, optionally together with nutrients for said microorganism, said culture having been produced by elevated pressure incubation.
We also describe a process for enhancing hydrocarbon recovery from a subterranean hydrocarbon reservoir which process involves injecting a fluid into said reservoir to drive the hydrocarbon to a producer well, wherein the composition of said fluid is preselected so as to inhibit or promote growth of microorganisms endogenous to said reservoir, e.g. on the basis of performance of the method of the invention.
The driving fluid used in this regard will typically be water, nitrogen, methane or carbon dioxide, preferably carbon dioxide. The composition of the fluid may be selected for example to include nutrients which cause the endogenous microorganisms to proliferate under aqueous conditions, i.e. so that biomass buildup will occur in aqueous zones of the reservoir causing the pores in the rock to clog and so reduce water flow to the producer well(s). Such nutrients may typically include water-soluble mineral salts.

Such processes will typically involve the injection conditions conventional for squeezes to effect well treatment and for fluid injection to enhance hydrocarbon recovery and thus need not be described in further detail.

Where the method of the invention yields novel, i.e. previously unknown, microorganisms, these also described herein as are rock-free compositions containing them. Such compositions may be dried (e.g. hydrophilized, optionally with a cryoprotective agent, e.g. a sugar) or may be liquid and may be under pressure or at ambient pressure. Where liquid, the solvent is preferably an organic solvent other than the native hydrocarbon in which the microorganism was found, e.g. a mineral oil or glycerol.

The method of the invention is typically effected in a fermentation reactor capable of operating under down-hole pressures. This is quite different from conventional fermentation reactors which are generally glass vessels or thin metal-skinned vessels. Such high pressure reactors are also described herein. We therefore also describe a microorganism fermentation reactor comprising an incubation chamber having an inlet port, and preferably also a sample removal port, the walls of said chamber being of a material sufficiently strong to withstand a pressure differential of at least 180 bar, more preferably at least 200 bar, especially at least 300 bar, more particularly up to 900 bar. The reactor described herein is preferably provided with a fermentation monitor and a heat source, as well as with valves capable of operation to reduce the pressure in the chamber slowly, e.g. at a rate of less than 50 bar/hour, preferably less than 10 bar/hour, more preferably less than 1 bar/hour. The heat source may typically be integral to the incubation chamber; however on a laboratory scale the reactor may simply be placed in an oven.

The down-hole sample may be collected at or near the point within the well at which the hydrocarbon enters. Devices capable of sampling in this way are known already. Preferably however the sample is taken from the pressurized fluid flow after it leaves the ground using a pressurized displacement cell. The pressurized displacement cell is then preferably insulated or place in a heated container to maintain the elevated temperature of the fluid sample before it is transferred to the fermentation reactor. The samples may if desired be taken from different flow-lines from the reservoir before they converge so that different microbial flora from different reservoir zones maybe detected.

Maintaining sample pressure from sample taking to transfer into the fermentation reactor is important as otherwise pressure-drop sensitive microorganisms will fragment and will not be grown in the reactor. Moreover maintaining pressure and temperature ensures the growth medium approximates to down-hole conditions in terms of dissolved gas content, etc.

The invention will now be described with reference to the accompanying drawings in which:
Figures 1, 5 and 6 are schematic drawings of reactors described herein;
Figures 2 and 3 are gel electrophoresis patterns for samples cultured using the reactor of Figure 1 and under control conditions; and
Figure 4 is a bar chart showing the numbers and types of microorganisms in cultures produced according to the method of the invention and in a control run..

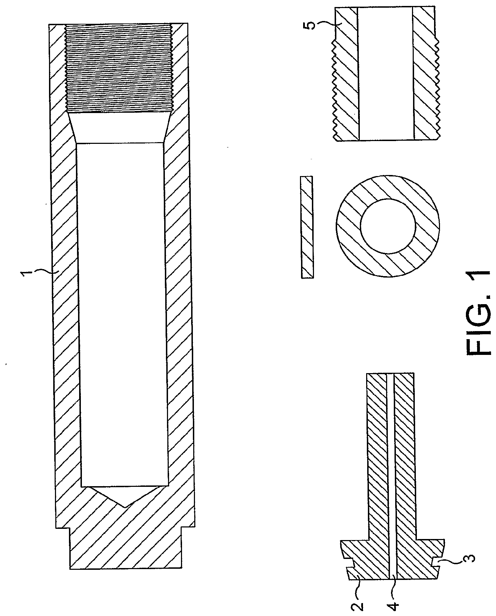

Referring to Figure 1 there is shown a horizontal cross-section of an open-ended cylinder 1. This is machined from 316 steel or Hastelloy C and is capable of withstanding pressures of 350 bar.

The open end of cylinder 1 may be closed with a tapered plug 2 which incorporates an O-ring in groove 3 and has a central vent 4 for fluid addition or removal. Plug 2 is held in place by a hollow threaded bolt 5 which engages with the internal threading at the mouth of cylinder 1. Preferably a washer (not shown) is placed between plug 2 and bolt 5 to minimize rotation of plug 2 while bolt 5 is tightened or loosened.

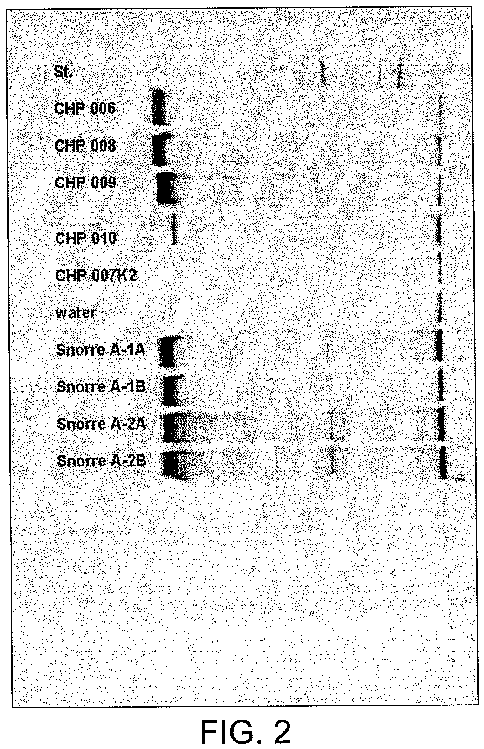

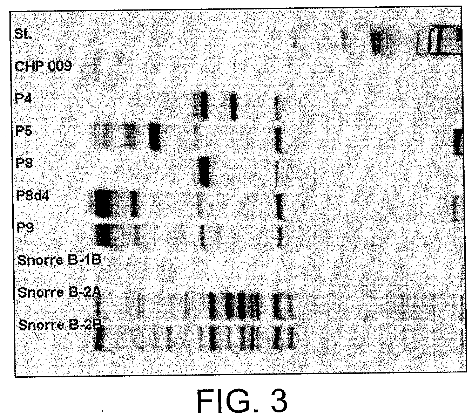

Referring to Figures 2 and 3 there are shown DGGE patterns for 21 cultures. Those designated CHP were of samples taken and cultured under reservoir conditions, except where the designation includes the letter K which is a control sample cultured at 1 bar. It will be seen that bacteria in CHP samples grew that did not grow in the CHP-K sample.

Figure 2 relates to test samples amplified using primers universal for eubacteria. Figure 3 relates to test samples amplified using primers universal for Achaea.

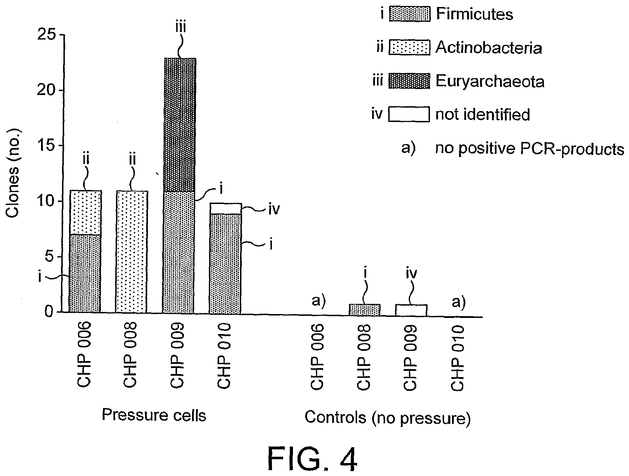

Figure 4 shows the number of clones produced culturing four different samples taken from reservoirs either at high pressure (Pressure cells) or at ambient pressure (Controls), and then carrying out PCR amplification on the resultant cultures.

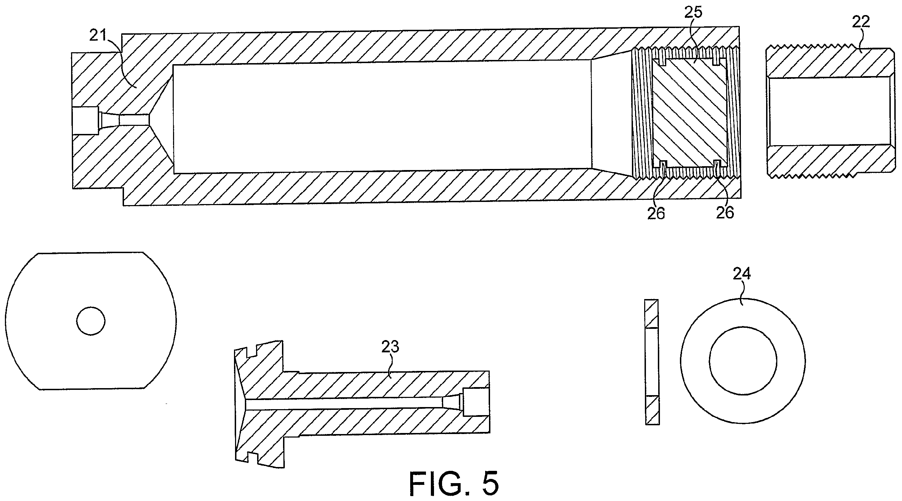

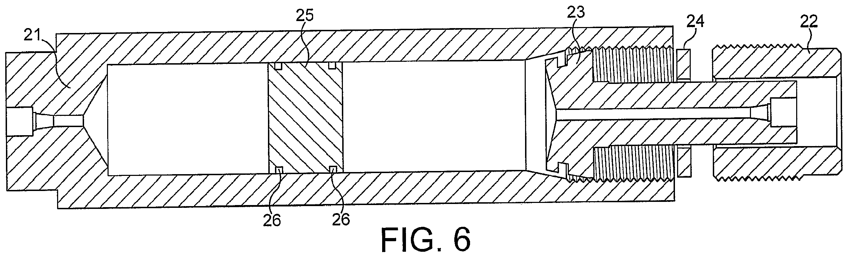

Figures 5 and 6 are, respectively, exploded and assembled schematics showing a cylinder 21 of 316 steel, a bolt 22 of JM7 (an aluminium bronze), a Teflon® plunger 23, a disk 24 of JM7, a plug 25 of 316 steel and Viton® O-rings 26.

## Claims

1. A method of producing a culture of subterranean microorganisms, said method comprising:
(i) obtaining a flow of hydrocarbon fluid containing microorganisms from a subterranean reservoir and taking a sample of said fluid after the flow has left the ground, wherein the pressure of the fluid flow at the point of sampling exceeds ambient pressure; and
(ii) while maintaining said sample at the pressure of the fluid flow at the point of sampling, transferring it into a fermentation reactor;
(iii) incubating said sample in said reactor at a pressure of 50-150% of that of the reservoir from which the sample was taken,
such that the conditions in the fermentation reactor promote culturing of the microorganisms contained within the hydrocarbon fluid sample.

2. The method of claim 1 wherein said sample is incubated in said reactor at a temperature of 60 to 160°C.

3. The method of claim 2, wherein the sample is incubated at a temperature of 80 to 100°C.

4. The method of any preceding claim wherein the sample is incubated at a pressure of 100 to 900 bar, preferably 230 to 280 bar.

5. The method of any preceding claim wherein incubation is effected by mixing the sample with a nutrient comprising at least pulverulent rock.

6. The method of any preceding claim wherein incubation is for from 7 to 360 days.

7. The method of any preceding claim further comprising bringing the culture to ambient pressure and temperature over at least 10 hours.

8. A process for subterranean reservoir treatment, which process comprises injecting a culture of a microorganism into said reservoir, optionally together with nutrients for said microorganism, said culture having been produced by elevated pressure incubation as defined in any one of claims 1 to 7.

9. The process of claim 8 wherein said subterranean reservoir is a hydrocarbon well.

10. A process for enhancing hydrocarbon recovery from a subterranean hydrocarbon reservoir which process involves injecting a fluid into said reservoir to drive the hydrocarbon to a producer well, wherein the composition of said fluid is preselected so as to inhibit or promote growth of microorganisms endogenous to said reservoir, said microorganisms having been identified on the basis of the method as defined in any one of claims 1 to 7.

## Patentansprüche

1. Verfahren zum Herstellen einer Kultur von unterirdischen Mikroorganismen, wobei das Verfahren umfasst:
(i) Erhalten eines Mikroorganismen enthaltenden Stroms von KohlenwasserstoffFluid aus einem unterirdischen Reservoir und Entnehmen einer Probe des Fluids, nachdem der Strom den Boden verlassen hat, wobei der Druck des Fluidstroms an der Stelle der Probeentnahme den Außendruck überschreitet; und
(ii) während des Haltens der Probe bei dem Druck des Fluidstroms an der Stelle der Probeentnahme diese in einen Fermentationsreaktor überführen;
(iii) Inkubieren der Probe in dem Reaktor bei einem Druck von 50 bis 150% desjenigen des Reaktors, von dem die Probe entnommen wurde,
so dass die Bedingungen in dem Fermentationsreaktor ein Kultivieren der in der Probe des Kohlenwasserstoff-Fluids enthaltenden Mikroorganismen fördern.

2. Verfahren nach Anspruch 1, wobei die Probe in dem Reaktor bei einer Temperatur von 60° bis 160 °C inkubiert wird.

3. Verfahren nach Anspruch 2, wobei die Probe bei einer Temperatur von 80° bis 100 °C inkubiert wird.

4. Verfahren nach einem der vorgehenden Ansprüche, wobei die Probe bei einem Druck von 100 bis 900 bar, bevorzugt 230 bis 280 bar, inkubiert wird.

5. Verfahren nach einem der vorgehenden Ansprüche, wobei die Inkubation durch Mischen der Probe mit einem mindestens ein feinpulveriges Gestein umfassenden Nährstoff herbeigeführt wird.

6. Verfahren nach einem der vorgehenden Ansprüche, wobei die Inkubation von 7 bis 360 Tage dauert.

7. Verfahren nach einem der vorgehenden Ansprüche, umfassend die Kultur über mindestens 10 Stunden auf Außendruck und -temperatur zu bringen.

8. Verfahren für unterirdische Reservoir-Behandlung, wobei das Verfahren Einspritzen einer Kultur eines Mikroorganismus in das Reservoir wahlweise zusammen mit Nährstoffen für den Mikroorganismus umfasst, wobei die Kultur durch Inkubation bei erhöhtem Druck nach einem der Ansprüche 1 bis 7 erzeugt worden ist.

9. Verfahren nach Anspruch 8, wobei das unterirdische Reservoir eine Kohlenwasserstoff-Bohrung ist.

10. Verfahren zum Erhöhen der Kohlenwasserstoffgewinnung aus einem unterirdischen Kohlenwasserstoff-Reservoir, wobei das Verfahren das Einspritzen eines Fluids in das Reservoir umfasst, um den Kohlenwasserstoff zu einem Produktionsbohrloch zu treiben, wobei die Zusammensetzung des Fluides vorausgewählt wird, um Wachstum von Mikroorganismen zu hemmen oder zu fördern, die in diesem Reservoir endogen sind, wobei die Mikroorganismen auf der Grundlage des in einem der Ansprüche 1 bis 7 festgelegten Verfahrens identifiziert worden sind.

## Revendications

1. Procédé de production d'une culture de micro-organismes souterrains, ledit procédé comprenant :
(i) l'obtention d'un écoulement de fluide d'hydrocarbure contenant des micro-organismes depuis un réservoir souterrain et la prise d'un échantillon dudit fluide après que l'écoulement a quitté le sol, dans lequel la pression de l'écoulement de fluide au niveau du point d'échantillonnage excède la pression ambiante ;
(ii) tout en maintenant ledit échantillon à la pression de l'écoulement de fluide au niveau du point d'échantillonnage, son transfert à l'intérieur d'un réacteur de fermentation ; et
(iii) l'incubation dudit échantillon dans ledit réacteur à une pression qui vaut 50 à 150 % celle du réservoir à partir duquel l'échantillon a été pris,
de telle sorte que les conditions dans le réacteur de fermentation favorisent la mise en culture des micro-organismes qui sont contenus à l'intérieur de l'échantillon de fluide d'hydrocarbure.

2. Procédé selon la revendication 1, dans lequel ledit échantillon est incubé dans ledit réacteur à une température de 60 à 160 °C.

3. Procédé selon la revendication 2, dans lequel l'échantillon est incubé à une température de 80 à 100 °C.

4. Procédé selon l'une quelconque des revendications qui précèdent, dans lequel l'échantillon est incubé à une pression de 100 à 900 bars, de préférence de 230 à 280 bars.

5. Procédé selon l'une quelconque des revendications qui précèdent, dans lequel l'incubation est réalisée en mélangeant l'échantillon avec un nutriment qui comprend au moins une roche pulvérulente.

6. Procédé selon l'une quelconque des revendications qui précèdent, dans lequel l'incubation dure de 7 à 360 jours.

7. Procédé selon l'une quelconque des revendications qui précèdent, comprenant en outre le fait d'amener la culture à pression et température ambiantes sur au moins 10 heures.

8. Procédé pour le traitement d'un réservoir souterrain, lequel procédé comprend l'injection d'une culture d'un micro-organisme à l'intérieur dudit réservoir, en option en association avec des nutriments pour ledit micro-organisme, ladite culture ayant été produite par incubation à pression élevée tel que défini selon l'une quelconque des revendications 1 à 7.

9. Procédé selon la revendication 8, dans lequel ledit réservoir souterrain est un puits d'hydrocarbure.

10. Procédé pour améliorer l'extraction d'hydrocarbure à partir d'un réservoir d'hydrocarbure souterrain, lequel procédé met en jeu l'injection d'un fluide à l'intérieur dudit réservoir de manière à entraîner l'hydrocarbure jusqu'à un puits de production, dans lequel la composition dudit fluide est présélectionnée de manière à inhiber ou à favoriser la croissance de micro-organismes qui sont endogènes par rapport audit réservoir, lesdits micro-organismes ayant été identifiés sur la base du procédé tel que défini selon l'une quelconque des revendications 1 à 7.
